# EUROPÄISCHE PATENTANMELDUNG

(11) **EP 4 763 797 A1**
(43) Veröffentlichungstag der Anmeldung: **24.06.2026**
(21) Anmeldenummer: 25202697.6
(22) Anmeldetag: 17.09.2025
(51) Int. Cl.: C01B 3/0005

(54) **VERFAHREN UND SYSTEM ZUR QUALITÄTSBESTIMMUNG EINES FLÜSSIGEN ORGANISCHEN WASSERSTOFFTRÄGERMATERIALS**

(30) Priorität: 17.12.2024 DE 102024212045
(71) Anmelder: HYDROGENIOUS LOHC TECHNOLOGIES GMBH, 91058 Erlangen (DE)
(72) Erfinder: Obermeier, Jonas, 91052 Erlangen (DE); Steinbach, Daniel, 97514 Oberaurach (DE); Kreissl, Hannah, 91052 Erlangen (DE)
(74) Vertreter: Rau, Schneck & Hübner Patentanwälte Rechtsanwälte PartGmbB

(57) **Zusammenfassung**

Ein Verfahren zur Qualitätsbestimmung eines flüssigen organischen Wasserstoffträgermaterials umfasst das Nutzen des flüssigen organischen Wasserstoffträgermaterials in einem LOHC-Anwendungssystem (1), das Messen mindestens zwei physikalischer Stoffeigenschaften des flüssigen organischen Wasserstoffträgermaterials in dem LOHC-Anwendungssystem (1), das Vergleichen der gemessenen physikalischen Stoffeigenschaften mit Kennwerten für das flüssige organische Wasserstoffträgermaterial bei unterschiedlich großen Hydriergraden und bei unterschiedlich großen Verunreinigungsproduktkonzentrationen sowie das Bestimmen des Hydriergrades (h) und einer ersten Verunreinigungsproduktkonzentration (c) als Qualitätskenngrößen des flüssigen organischen Wasserstoffträgermaterials.

## Beschreibung

Der Inhalt der deutschen Patentanmeldung DE 10 2024 212 045.6 wird durch Bezugnahme hierin aufgenommen.

Die Erfindung betrifft ein Verfahren und ein System zur Qualitätsbestimmung eines flüssigen organischen Wasserstoffträgermaterials.

Flüssiges organisches Wasserstoffträgermaterial, das aufgrund seiner englischen Bezeichnung "Liquid Organic Hydrogen Carrier" auch kurz als LOHC bezeichnet wird, dient zur reversiblen Speicherung und Freisetzung von Energie in Form von Wasserstoff. Für die Nutzung von LOHC in der Praxis beispielsweise zur Überprüfung des Verbrauchs für Abrechnungszwecke und/oder zur Regelung von Prozessen und/oder Komponenten ist die Qualitätsbestimmung des LOHC von Bedeutung. Als Qualitätskenngrößen können der Hydriergrad und die Reinheit des LOHC dienen. Der Hydriergrad definiert einen Beladezustand des LOHC. Wenn das LOHC vollständig beladen ist, beträgt der Hydriergrad 100 %. Wenn das LOHC vollständig entladen ist, beträgt der Hydriergrad 0 %. In der Praxis ist der Hydriergrad kleiner als 100 %, aber größer als 0 %. Die Reinheit des LOHC kann anhand eines Anteils von Nebenprodukten und/oder Verunreinigungen quantifiziert werden. Nebenprodukte und Verunreinigungen werden im Sinne der Patentanmeldung unter der Bezeichnung Verunreinigungsprodukte zusammengefasst.

Der Hydriergrad kann im Labor mittels Kernspinresonanzspektroskopie, die auch als NMR-Spektroskopie bezeichnet wird, und/oder mittels Gaschromatographie bestimmt werden. Diese Bestimmung ist zeitaufwändig und nur offline durchführbar. Für eine Hydriergradbestimmung inline, also in einem System, ist auf Basis von Musterproben von beladenem und entladenem LOHC möglich, wobei für die Musterproben zusätzlich physikalische Stoffeigenschaften, insbesondere Dichte und/oder Brechzahl, berechnet und in Verbindung mit dem Hydriergrad eine Korrelation erstellt werden. Die Brechzahl wird auch als Brechungsindex bezeichnet. Dichte und/oder Brechzahl können inline gemessen und entsprechend der Hydriergrad abgeschätzt werden. Diese inline-Bestimmung ist ungenau, insbesondere da die Korrelation zwischen Hydriergrad und Dichte und/oder Brechzahl temperaturabhängig ist. Darüber hinaus haben Verunreinigungsprodukte einen Einfluss auf die Korrelation von Hydriergrad und Dichte und/oder Brechzahl. Da in dem LOHC-Anwendungssystem in der Praxis der Grad der Verunreinigung unbekannt ist, weist diese inline-Messung einen nicht bestimmbaren, systematischen Fehler auf.

Der Messaufbau für die Bestimmung von Verunreinigungsprodukten ist komplex und für eine inline-Messung nicht anwendbar.

Der Erfindung liegt die Aufgabe zugrunde, die Qualitätsbestimmung von LOHC zu verbessern, insbesondere zu vereinfachen und eine inline-Durchführung der Qualitätsbestimmung zu ermöglichen.

Diese Aufgabe ist erfindungsgemäß gelöst, durch ein Verfahren mit den Merkmalen des Anspruchs 1 sowie durch ein System mit den Merkmalen des Anspruchs 11.

Der Kern der Erfindung besteht darin, dass der Hydriergrad und eine erste Verunreinigungsproduktkonzentration als Qualitätskenngrößen des LOHC vereinfacht bestimmt werden können, indem ein Vergleich von gemessenen physikalischen Stoffeigenschaften mit entsprechenden Kennwerten für LOHC durchgeführt wird. Wesentlich ist, dass mindestens zwei physikalische Stoffeigenschaften des LOHC gemessen werden. Das Messen mehrerer physikalischer Stoffeigenschaften umfasst also auch das Messen von nur einer einzigen physikalischen Stoffeigenschaft bei verschiedenen Messbedingungen, insbesondere bei verschiedenen Temperaturen. Die Messung der physikalischen Stoffeigenschaften erfolgt insbesondere unmittelbar.

Insbesondere erfolgt das Messen in einem LOHC-Anwendungssystem, also inline. Als LOHC-Anwendungssystem wird ein System verstanden, in dem LOHC bereitgestellt und insbesondere genutzt wird. Ein LOHC-Anwendungssystem umfasst insbesondere einen Hydrierreaktor, der zum Hydrieren, also Beladen, des LOHC mit Wasserstoff dient. Das LOHC-Anwendungssystem umfasst insbesondere einen Dehydrierreaktor, der zum Dehydrieren, also Entladen, des LOHC dient. Mit dem Dehydrierreaktor kann Wasserstoffgas von dem LOHC freigesetzt werden. Das LOHC-Anwendungssystem umfasst insbesondere auch Lagerstätten und/oder Umschlagstätten, an welchen das LOHC gelagert und/oder umgeschlagen wird. Insbesondere findet an den Lager- und Umschlagstätten keine Hydrierung und/oder Dehydrierung statt. Eine Lager- oder Umschlagstätte ist beispielsweise ein Hafen. Insbesondere ist das LOHC-Anwendungssystem ein Kreislaufsystem, in dem das LOHC im Sinne eines geschlossenen Materialkreislaufs genutzt werden kann. Das LOHC-Anwendungssystem umfasst insbesondere auch mindestens einen Speicherbehälter und/oder Fluidleitungen. Das LOHC-Anwendungssystem kann aber auch nur teilweise einen geschlossenen Kreislauf abbilden, also beispielsweise einen Dehydrierreaktor mit fluidtechnisch damit verbundenem Speicherbehälter und einer Wasserstoffverwertungseinheit. Die Wasserstoffverwertungseinheit kann eine Brennstoffzelle für die Verstromung des freigesetzten Wasserstoffgases sein. Alternativ kann der freigesetzte Wasserstoff auch in einem chemischen Prozess genutzt oder in eine Wasserstoffgasleitung für den Transport eingespeist werden. Insbesondere kann das LOHC-Anwendungssystem als geschlossener Kreislauf ausgebildet sein, wobei Abschnitte des geschlossenen Kreislaufes an unterschiedlichen Orten angeordnet sind. Die entsprechenden Abschnitte können beispielsweise durch LOHC-Transportfahrzeuge miteinander verbunden werden.

Wesentlich ist weiterhin, dass der Vergleich der gemessenen physikalischen Stoffeigenschaften mit den Kennwerten bei unterschiedlich großen, also verschiedenen, aber definierten Hydriergraden sowie bei unterschiedlich großen, also verschiedenen, aber definierten, Verunreinigungsproduktkonzentrationen erfolgt. Insbesondere wird die Konzentration für ein Verunreinigungsprodukt gezielt bestimmt. Eine Verunreinigungsproduktkonzentration betrifft die Konzentration eines Verunreinigungsprodukts im LOHC. Unterschiedlich große Verunreinigungsproduktkonzentrationen liegen beispielsweise vor, wenn für ein Verunreinigungsprodukt unterschiedliche Konzentrationswerte, also beispielsweise 1 % und 5 % betragen. Dazu ist es von Vorteil, wenn die für den Vergleich genutzten Kennwerte insbesondere in Form eines Kennlinienfeldes und/oder als Kennwerttabelle vorliegen. Die Kennwerte können, insbesondere vorab, berechnet werden. Insbesondere werden die Kennwerte basierend auf gemessenen Stützwerten interpoliert.

Verunreinigungsprodukte im Sinne der Erfindung sind Verbindungen oder Elemente, die sich von dem genutzten flüssigen organischen Wasserstoffträgermaterial unterscheiden. Ein Verunreinigungsprodukt kann ein flüssiges organisches Wasserstoffträgermaterial sein, aber jedenfalls ein anderes als das in dem LOHC-Anwendungssystem genutzte. Wenn beispielsweise das genutzte Wasserstoffträgermaterial Benzyltoluol ist, können Dibenzyltoluol und/oder Methylfluoren als Wasserstoffträgermaterial Verunreinigungsprodukte sein.

Das Verunreinigungsprodukt kann eine Verunreinigung sein, die insbesondere initial in dem Wasserstoffträgermaterial enthalten ist. Eine Verunreinigung umfasst insbesondere ein Molekül, ist insbesondere aus diesem Molekül gebildet, das bei der Hydrierung und/oder Dehydrierung von LOHC nicht hydriert und/oder dehydriert wird. Insbesondere ist eine Verunreinigung eine anorganische Verbindung, insbesondere Wasser. Verunreinigungen können auch Verbindungen umfassen, die ein Wasserstoffträgermaterial sind oder daraus gebildet worden sind. Verunreinigungen sind insbesondere bereits enthalten, wenn das LOHC in dem LOHC-Anwendungssystem erstmals genutzt wird. Verunreinigungen werden beispielsweise bei der Herstellung des LOHC gebildet und/oder werden beispielsweise durch externe Quellen in das LOHC-Anwendungssystem eingebracht, insbesondere beim Transport und/oder der Lagerung.

Verunreinigungsprodukte können auch Nebenprodukte sein, die bei der Nutzung des LOHC im LOHC-Anwendungssystem gebildet werden, insbesondere bei der Hydrierung und/oder Dehydrierung von LOHC.

Verunreinigungsprodukte sind insbesondere artgleich zu LOHC, sind also insbesondere Kohlenwasserstoffverbindungen, insbesondere heteroatomfrei. Verunreinigungsprodukte können aber auch artfremd sein und/oder Heteroatome aufweisen. Beispiele für Verunreinigungsprodukte sind insbesondere Methylfluoren oder hydrierte Spezies davon, Diphenylmethan, Toluol, Methylcyclohexan, und/oder Dibenzyltoluol.

Der Hydriergrad und die erste Verunreinigungsproduktkonzentration können insbesondere dadurch bestimmt werden, dass die korrespondierenden Werte aus den ermittelten Kennwerten abgelesen und/oder berechnet werden, insbesondere durch Interpolation oder Extrapolation, insbesondere lineare Interpolation und/oder lineare Extrapolation.

Die Ermittlung der Kennwerte beruht auf einem Mustersystem des LOHC, das gezielt mit unterschiedlich großen Hydriergraden bereitgestellt wird, und insbesondere mit genau einem Verunreinigungsprodukt, dessen Konzentration in dem LOHC gezielt veränderlich eingestellt wird.

Das Mustersystem kann auch mehrere Verunreinigungsprodukte aufweisen. Voraussetzung ist, dass die zu bestimmenden Kennwerte für die Ausgangsstoffe, also das LOHC, bei verschiedenen Hydriergraden und des Verunreinigungsprodukts oder der Verunreinigungsprodukte bekannt sind. Für die Berechnung der Kennwerte werden fiktive Mischungen betrachtet. Je mehr Stützwerte für das Kennlinienfeld existieren, desto genauer ist die Berechnung der Kennwerte für die fiktiven Mischungen. Die Berechnung der Kennwerte zwischen den Stützwerten basiert insbesondere auf einer linearen Interpolation und geht insofern von einer idealen Mischung aus. Unter einer idealen Mischung wird verstanden, dass keine Volumeneffekte auftreten, so dass die Volumina der Reinstoffe, die gemischt worden sind, addiert werden können. Je mehr Stützwerte im Kennlinienfeld existieren, desto genauer werden auch nicht-lineare Zusammenhänge abgebildet.

Ausgehend von diesen Annahmen und den bekannten Stoffdaten kann für das Mustersystem für jeden beliebigen Mischzustand die jeweiligen physikalischen Stoffeigenschaften berechnet werden. Vorteilhaft ist es, wenn Kennwerte für Mischungszustände ermittelt werden, bei denen jeweils die verschiedenen physikalischen Stoffeigenschaften konstant sind. Die Kennwerte können vorteilhaft als Kennlinien, insbesondere in einem Kennlinienfeld, als Iso-Linien dargestellt werden, entlang der die jeweilige physikalische Stoffeigenschaft konstant ist.

Eine wesentliche Erkenntnis der Erfindung beruht darauf, dass für das in dem LOHC-Anwendungssystem bereitgestellte LOHC sowohl der Hydriergrad als auch die mindestens eine Verunreinigungsproduktkonzentration unbekannt sind. Diese beiden Qualitätskenngrößen können aber bestimmt werden, wenn zwei Messgrößen gemessen und ausgewertet werden, insbesondere wenn es sich um voneinander unabhängige Messgrößen handelt. Es wurde gefunden, dass als unabhängige Messgrößen physikalische Stoffeigenschaften dienen.

Wesentlich ist, dass die Anzahl der zu messenden physikalischen Stoffeigenschaften mindestens so groß ist, wie die Anzahl der zu bestimmenden Qualitätskenngrößen. Wesentlich ist auch, dass die verschiedenen physikalischen Stoffeigenschaften voneinander unabhängig sind, sich also gegenseitig nicht beeinflussen.

Das erfindungsgemäße Verfahren ist unkompliziert, weil die physikalischen Stoffeigenschaften des LOHC unmittelbar und direkt mit an sich bekannten Messgeräten gemessen werden können. Insbesondere können die physikalischen Stoffeigenschaften inline, also in dem LOHC-Anwendungssystem, gemessen werden. Insbesondere erfolgt die Messung der physikalischen Stoffeigenschaften in einer Komponente des LOHC-Anwendungssystems, insbesondere während eines Hydrierprozesses, insbesondere in dem Hydrierreaktor, insbesondere während eines Dehydrierprozesses, in dem Dehydrierreaktor, in einem Speicherbehälter und/oder in Fluidleitungen, also inline. Die Messung kann auch online erfolgen, insbesondere in einer Abzweigleitung, die auch als Bypass-Leitung bezeichnet wird.

Sowohl die Inline-Messung als auch die Online-Messung können kontinuierlich erfolgen. Die Bestimmung von Hydriergrad und erster Verunreinigungsproduktkonzentration als Qualitätskenngrößen ist genau, weil die für den Vergleich genutzten Kennwerte unterschiedliche Hydriergrade und unterschiedliche Verunreinigungsproduktkonzentrationen berücksichtigen.

Ein Verfahren gemäß Anspruch 2 ermöglicht die Bestimmung einer weiteren, nämlich dritten, Qualitätskenngröße, insbesondere einer zweiten Verunreinigungsproduktkonzentration. Durch Messen einer dritten physikalischen Stoff-eigenschaft, die insbesondere unabhängig von den beiden ersten physikalischen Stoffeigenschaften ist, kann die dritte Unbekannte entsprechend bestimmt werden. Dazu ist erforderlich, dass entsprechende Kennwerte auch für die zweite Verunreinigungsproduktkonzentration, insbesondere vorab, bestimmt und/oder berechnet worden sind. Die zweite Verunreinigungsproduktkonzentration unterscheidet sich von der ersten Verunreinigungsproduktkonzentration dadurch, dass es sich um ein anderes Verunreinigungsprodukt handelt.

Es versteht sich, dass weitere physikalische Stoffeigenschaften gemessen werden können, um weitere Qualitätskenngrößen zu bestimmen und/oder die Messgenauigkeit des Hydriergrades und der ersten Verunreinigungsproduktkonzentration zu erhöhen. Bei den weiteren Qualitätskenngrößen kann es sich insbesondere um weitere Verunreinigungsproduktkonzentrationen handeln. Eine Erhöhung der Messgenauigkeit ist beispielsweise dadurch möglich, dass eine erste und zweite physikalische Stoffeigenschaft zur Bestimmung der Qualitätskenngrößen in einem ersten Konzentrationsbereich und die zweite und eine dritte physikalische Stoffeigenschaft für die Bestimmung der Qualitätskenngrößen in einem anderen, insbesondere angrenzenden zweiten Konzentrationsbereich dienen.

Ein Verfahren gemäß Anspruch 3 ermöglicht eine erhöhte Messgenauigkeit, indem insbesondere die Empfindlichkeit der physikalischen Stoffeigenschaften ausgehend von Reinstoffdaten auf das Mischungsverhältnis besonders groß ist.

Ein Verfahren gemäß Anspruch 4 verbessert die Inline-Messung der physikalischen Stoffeigenschaften. Ein, insbesondere geringer, Teilstrom des LOHC kann mittels mindestens einer Bypass-Leitung entlang einer Fluidleitung, insbesondere parallel zu einer Fluidleitung, abgezweigt und zu Messzwecken genutzt werden. Die Messung ist dadurch verbessert, insbesondere die Qualität der Messergebnisse erhöht. Insbesondere ist keine Störung der Fluidströmung zu erwarten. Entlang der Bypass-Leitung tritt ein reduzierter Massenstrom auf. Der reduzierte Massenstrom ist insbesondere unkomplizierter und/oder schneller anpassbar, insbesondere an verschiedene Messbedingungen, insbesondere verschiedene Messtemperaturen. Insbesondere wird der Brechungsindex n aufgrund seiner ausgeprägten Temperaturabhängigkeit in einer, insbesondere temperierten, Bypass-Leitung gemessen. Zusatzaufwand insbesondere für das Heizen und/oder Kühlen der Stoffströme ist reduziert und insbesondere vermieden. Ein zusätzlicher Vorteil der Bypass-Leitung ist die verbesserte oder grundsätzlich ermöglichte Zugänglichkeit einer Messeinrichtung direkt im Fluidstrom, der in den verschiedenen Komponenten des LOHC-Anwendungssystems nicht ohne Weiteres gegeben ist. Dadurch werden Bereiche in dem LOHC-Anwendungssystem für die Messungen vorteilhaft erschlossen.

In einer Ausgestaltung können zumindest teilweise die physikalischen Stoffeigenschaften, insbesondere eine physikalische Stoffeigenschaft, insbesondere mehrere physikalische Eigenschaften und insbesondere nicht sämtliche physikalische Stoffeigenschaften, in mindestens einer Bypass-Leitung gemessen werden.

Zusätzlich oder alternativ können mehrere Bypass-Leitungen vorhanden sein. Beispielsweise können die Stoffströme in den Bypass-Leitungen unterschiedlich temperiert werden, wobei in jeder Bypass-Leitung die gleiche Stoffeigenschaft gemessen wird. In diesem Fall wird also nur eine physikalische Stoffeigenschaft in mehreren Bypass-Leitungen jeweils gemessen.

Ein Verfahren gemäß Anspruch 5 hat sich für die Realisierung als besonders vorteilhaft erwiesen. Unterschiedliche, insbesondere unabhängige, physikalische Stoffeigenschaften ermöglichen ein Messergebnis mit hoher Messqualität. Entsprechend können die Qualitätskenngrößen mit hoher Genauigkeit bestimmt werden. Als besonders vorteilhaft haben sich Dichte und Brechungsindex für die physikalischen Stoffeigenschaften erwiesen. Die Dichte eignet sich für die Anwendung der linearen Mischung gemäß einer gravimetrischen Zusammensetzung bei der Ermittlung der Stoffdaten. Die Dichte weist zudem eine ausgeprägte Temperaturabhängigkeit auf, so dass die physikalische Stoffeigenschaft auch bei unterschiedlichen Temperaturen gemessen werden kann. Die Messung der Dichte kann beispielsweise mit einem Messverfahren, beruhend auf dem Coriolis-Prinzip, insbesondere mit einem Coriolis-Massendurchflussmesser, erfolgen, insbesondere im Hauptstrom. Die Dichte kann aber auch radiometrisch oder beruhend auf anderen Prinzipien wie der direkt von der Dichte abhängigen charakteristischen Schwingfrequenz, dem sogenannten Biegeschwinger-Prinzip, erfolgen.

Der Brechungsindex ermöglicht die Anwendung der linearen Mischungsregel gemäß der molaren Zusammensetzung. Auch der Brechungsindex weist eine ausgeprägte Temperaturabhängigkeit auf. Der Brechungsindex kann mittels eines Refraktometers gemessen werden.

Zusätzlich oder alternativ können als physikalische Stoffeigenschaften auch die spezifische Wärmekapazität, die Leitfähigkeit, insbesondere die Wärmeleitfähigkeit, die dynamische Viskosität und/oder die Schallgeschwindigkeit dienen. Die spezifische Wärmekapazität eignet sich gemäß der linearen Mischungsregel gemäß der gravimetrischen Zusammensetzung. Die spezifische Wärmekapazität ist temperaturabhängig und kann mittels kalorimetrischer Messung mittels eines Sensors mit integriertem Heizkreis und Temperaturmessung ermittelt werden. Die Wärmeleitfähigkeit ist für die lineare Mischungsregel gemäß gravimetrischer Zusammensetzung geeignet. Die Wärmeleitfähigkeit weist eine geringe Temperaturabhängigkeit auf und kann mittels Sensor mit integriertem Heizkreis und Temperaturmessung gemessen werden. Die dynamische Viskosität eignet sich für die lineare Mischungsregel gemäß der gravimetrischen Zusammensetzung. Die dynamische Viskosität weist eine hohe Temperaturabhängigkeit auf und kann mittels eines Schwingquarzes gemessen werden. Für die Bestimmung des Hydriergrades ist die Linearität gegeben. Die Schallgeschwindigkeit ist temperatur- und druckabhängig und außerdem abhängig von der Dichte der zu messenden Substanz. Die Schallgeschwindigkeit ist für die lineare Mischungsregel gemäß gravimetrischer Zusammensetzung geeignet. Die Messung erfolgt durch die Bestimmung der Schalllaufzeit im zu untersuchenden Medium, welche für einen definierten Abstand zwischen Ultraschallquelle und -empfänger gemessen wird.

Ein Verfahren gemäß Anspruch 6 ist mit reduziertem Messaufwand durchführbar. Ein derartiges Verfahren ist anlagentechnisch unkompliziert und kostenunaufwändig. Es wurde gefunden, dass es ausreicht, nur eine physikalische Stoffeigenschaft zu messen, wenn sie eine ausgeprägte Temperaturabhängigkeit aufweist. In diesem Fall kann ein und dieselbe physikalische Stoffeigenschaft bei unterschiedlichen, insbesondere definierten, Temperaturen gemessen werden. Dadurch ist es möglich, zwei unabhängige Kennwerte mit nur einer einzigen physikalischen Stoffeigenschaft zu bestimmen. Das bedeutet, dass der Temperatureinfluss die Wirkung einer zusätzlichen physikalischen Stoffeigenschaft hat.

Ein Verfahren gemäß Anspruch 7 ist besonders anwendungsorientiert. Benzyltoluol ist ein LOHC, das für die Nutzung in einem LOHC-Anwendungssystem gut geeignet ist.

Grundsätzlich gilt, dass als LOHC eine Kohlenwasserstoffverbindung dient, die insbesondere ohne Hetero-Atome ausgeführt ist. Zusätzlich oder alternativ zu Benzyltoluol (BT) kann als LOHC in der zumindest teilweise entladenen Form auch Toloul, Dibenzyltoluol (DBT) oder Diphenylmethan dienen.

Die Berücksichtigung eines Verunreinigungsprodukts gemäß Anspruch 8 ist realitätsnah. Polycyclische Ringsysteme, insbesondere Kohlenwasserstoffe, in aromatischer oder aliphatischer Form unter anderem mit Methylgruppen als funktionelle Gruppen, besonders Methylfluoren, sind häufig auftretende Verunreinigungsprodukte bei der Hydrier- oder Dehydrierreaktion von Benzyltoluol. Es können weitere Verunreinigungsprodukte enthalten sein, die einen Ring, insbesondere aromatisch oder aliphatisch, mit optional entsprechenden funktionellen Gruppen enthalten. Beispiele hierfür sind Benzol, Toluol und Methylcyclohexan.

Ein Verfahren gemäß Anspruch 9 ermöglicht die Berechnung der Verunreinigungsproduktmenge, insbesondere als absoluten Wert. Für die Bestimmung dient insbesondere eine Strömungsmesstechnik, insbesondere ein Massedurchflussmesser. Mit der Strömungsmesstechnik kann die Konzentration des Verunreinigungsprodukts lokal zeitlich aufgelöst erfasst werden. Daraus kann die Menge des Verunreinigungsprodukts, insbesondere als Absolutwert, berechnet werden. Dies betrifft beispielsweise die Masse von Methylfluoren, insbesondere in einem Speicherbehälter. Zusätzlich ist es vorteilhaft, die Gesamtmasse unterschiedlicher Substanzen in dem LOHC-Fluidstrom zu ermitteln. Die Gesamtmasse der Substanzen kann insbesondere als Qualitätskenngröße für den Betrieb und insbesondere die Regelung des LOHC-Anwendungssystems dienen.

Ein Verfahren gemäß Anspruch 10 ermöglicht eine vorteilhafte Anwendung und Nutzung der Qualitätskenngrößen. Insbesondere können die Qualitätskenngrößen als Prozessregelgrößen dienen.

Insbesondere können das Hydrieren und/oder Dehydrieren in Abhängigkeit der Prozesstemperatur und/oder der LOHC-Verweilzeit im Reaktor so geregelt werden, dass der sich einstellende Hydriergrad und/oder die Verunreinigungsproduktbildung optimiert werden. Zusätzlich oder alternativ kann die Vorbereitung des LOHC auf die Hydrierung und/oder Dehydrierung geregelt werden, insbesondere ein Entgasen und/oder Trocknen. Insbesondere ist eine Korrelation von Verunreinigungsproduktkonzentration im LOHC und in dem bei der Dehydrierung freigesetzten Wasserstoff möglich. In diesem Fall sind Anpassungen an Parametern im freigesetzten Wasserstoffgasstrom zur Prozessregelung möglich. Als Prozesszustand wird insbesondere der Katalysatorzustand verstanden.

Die Qualitätskenngrößen können auch zur Überwachung der Katalysatorstabilität dienen, insbesondere bei der Hydrierung und/oder Dehydrierung. Es wurde erkannt, dass der Grad der Verunreinigungsprodukte signifikante Auswirkungen auf die Katalysatorstabilität hat, insbesondere bei der Hydrierung. Insbesondere besteht das Risiko, dass keine konstante, insbesondere keine reproduzierbare, Qualität des LOHC erzeugt werden kann, woraus unterschiedliche Hydriergrade und/oder unterschiedliche Verunreinigungsprodukte resultieren können. Deshalb ist eine Qualitätsbestimmung vorteilhaft beispielsweise bei einer Anlieferung von zumindest teilweise entladenem LOHC stromaufwärts eines Hydrierreaktors, insbesondere in einem vorgelagerten Behälter, und/oder in einem Zuführ-Fluidstrom vor dem Hydrierreaktor und insbesondere nach dem Behälter.

Zusätzlich oder alternativ können die Qualitätskenngrößen auch zur Qualitätskontrolle in der LOHC-Logistik dienen. Insbesondere kann dies an einer Übergabestelle zwischen zwei Logistikdienstleistern erfolgen, also beispielsweise von zwei Transportdienstleistern, insbesondere bei der Übergabe von LOHC von einem Transportschiff auf einen Transportzug und/oder einen Transport-Lkw und/oder bei einer Übergabe von einem Transportschiff und einem Speicherbehälter. Diese Qualitätskontrolle ist insbesondere deshalb vorteilhaft, weil sich die LOHC-Qualität bei Luftkontakt verändern kann, indem insbesondere weitere Verunreinigungsprodukte gebildet werden können.

Die Nutzung der Qualitätskenngrößen kann auch für die Bewertung des Katalysatorzustandes dienen. Dies kann beispielsweise dadurch erfolgen, dass der erste Reaktionshub, der als Differenz zwischen Eingangshydriergrad und Ausgangshydriergrad berechnet wird, bei definierten Bedingungen als Referenz für die Katalysatoraktivität, insbesondere dessen Deaktivierungsrate, überprüft werden kann. Zudem lässt die Bildung von Verunreinigungsprodukten eine Aussage über die Selektivität des Katalysators zu. Insbesondere kann die Menge neu gebildeter Verunreinigungsprodukte aus der Differenz der Verunreinigungsprodukte vor dem Reaktor zu den Verunreinigungsprodukten nach dem Reaktor berechnet werden. Die Analyse des Hydriergrades und der Verunreinigungsproduktkonzentrationen vor und nach dem Reaktor ermöglicht eine Aussage über den Zustand des Katalysators, insbesondere dessen Aktivität. Über den zeitlichen Verlauf dieser Zustandsbeschreibung können weitere Maßnahmen vorhergesagt werden wie beispielsweise die Notwendigkeit der Katalysatorregeneration und/oder der Katalysatoraustausch, indem insbesondere die abnehmende Katalysatoraktivität extrapoliert wird.

Darüber hinaus wurde gefunden, dass die Messung der Konzentration der Verunreinigungsprodukte in der Flüssigkeit eine Indikation über die Gasphasenqualität zulässt und insbesondere zur Bestimmung der Gasphasenqualität dienen kann, also insbesondere des Wasserstoffgases im Dehydrierprozess.

Ein System gemäß Anspruch 11 weist im Wesentlichen die Vorteile des Verfahrens gemäß Anspruch 1 auf, worauf hiermit verwiesen wird.

Ein System gemäß Anspruch 12 ermöglicht die vorteilhafte Nutzung der Qualitätskenngrößen für den Betrieb des LOHC-Anwendungssystems.

Sowohl die in den Patentansprüchen angegebenen Merkmale als auch die in dem Ausführungsbeispiel eines erfindungsgemäßen Systems angegebenen Merkmale sind jeweils für sich alleine oder in Kombination miteinander geeignet, den erfindungsgemäßen Gegenstand weiterzubilden. Die jeweiligen Merkmalskombinationen stellen hinsichtlich der Weiterbildungen des Erfindungsgegenstandes keine Einschränkung dar, sondern weisen im Wesentlichen lediglich beispielhaften Charakter auf.

Weitere Merkmale, Vorteile und Einzelheiten der Erfindung ergeben sich aus der nachfolgenden Beschreibung eines Ausführungsbeispiels anhand der Zeichnung.

Es zeigen:
- Fig. 1: eine schematische Darstellung eines erfindungsgemäßen Systems zur Qualitätsbestimmung eines LOHC,
- Fig. 2: ein Diagramm in Form eines Kennlinienfeldes zur Darstellung der Abhängigkeit des Hydriergrades und einer Verunreinigungsproduktkonzentration für Methylfluoren anhand der gemessenen physikalischen Stoffeigenschaften Dichte und Brechungsindex.

Ein als Ganzes mit 1 gekennzeichnetes LOHC-Anwendungssystem dient zur Nutzung von LOHC zum reversiblen Speichern und Freisetzen von Energie in Form von Wasserstoff. Das LOHC-Anwendungssystem 1 umfasst einen ersten Speicherbehälter 2, in dem beladenes LOHC, das als LOHC-H bezeichnet wird, bevorratet ist. Der erste Speicherbehälter 2 ist an einen Dehydrierreaktor 3 angeschlossen, der eine Entladeeinheit zum Freisetzen von Wasserstoff aus LOHC-H darstellt.

Der Dehydrierreaktor 3 ist mit einem zweiten Speicherbehälter 4 verbunden, in dem das in dem Dehydrierreaktor 3 entladene LOHC, das als LOHC-D bezeichnet wird, bevorratet werden kann.

An den Dehydrierreaktor 3 ist ferner eine Wasserstoffverwertungseinheit 5 angeschlossen, in der das in dem Dehydrierreaktor 3 freigesetzte Wasserstoffgas verwertet werden kann. Die Wasserstoffverwertungseinheit 5 ist insbesondere eine Brennstoffzelle.

Der zweite Speicherbehälter 4 und eine Wasserstoffquelle 6 sind an einen Hydrierreaktor 7 angeschlossen, der eine Beladeeinheit zum Beladen des LOHC-D mit Wasserstoff bildet. Von der Wasserstoffquelle 6 aus kann Wasserstoffgas zum Hydrieren des LOHC-D dem Hydrierreaktor 7 zugeführt werden.

Der Hydrierreaktor 7 ist mittels einer Fluidleitung mit dem ersten Speicherbehälter 2 verbunden.

Das LOHC-Anwendungssystem 1 gemäß dem gezeigten Ausführungsbeispiel stellt ein geschlossenes Kreislaufsystem dar.

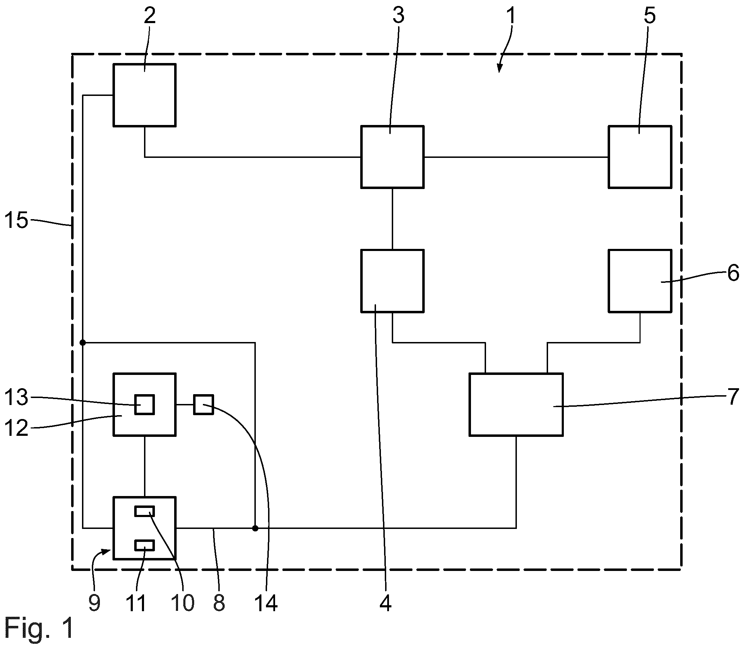

Es ist möglich, dass ein LOHC-Anwendungssystem 1 kein vollständig geschlossenes Kreislaufsystem darstellt und insbesondere nur eine oder einige der in Fig. 1 gezeigten Komponenten umfasst. Beispielsweise ist es denkbar, in einem ersten Teilsystem den ersten Speicherbehälter 2, den Dehydrierreaktor 3, den zweiten Speicherbehälter 4 und die Wasserstoffverwertungseinheit 5 anzuordnen. Der Hin- und Abtransport von LOHC-H bzw. LOHC-D kann über entsprechende Schnittstellen, die in Fig. 1 nicht dargestellt sind, erfolgen, insbesondere Transportleitungen und/oder Transportfahrzeuge wie Transportschiffe, Transportzüge und/oder Transport-Lastkraftwagen. Ein zweites Teilsystem könnte beispielsweise gebildet sein durch den zweiten Speicherbehälter 4, die Wasserstoffquelle 6, den Hydrierreaktor 7 und den ersten Speicherbehälter 2.

In dem LOHC-Anwendungssystem 1, insbesondere entlang einer Bypass-Fluidleitung 8 ist eine Messvorrichtung 9 angeordnet, die einen ersten Sensor 10 und einen zweiten Sensor 11 aufweist. Die Sensoren 10, 11 dienen zum Messen physikalischer Stoffeigenschaften des LOHC. Insbesondere ist der erste Sensor 10 als Coriolis-Massendurchflussmesser ausgeführt. Der erste Sensor 10 dient zur Dichtemessung des LOHC. Insbesondere ist der zweite Sensor 11 als Refraktometer zur Messung des Brechungsindex des LOHC ausgeführt.

Die Messvorrichtung 9 ist insbesondere stromaufwärts des ersten Speicherbehälters 2 und insbesondere stromaufwärts des Dehydrierreaktors 3 angeordnet. Die Messvorrichtung 9 kann auch zwischen dem ersten Speicherbehälter 2 und dem Dehydrierreaktor 3 angeordnet sein. Insbesondere können auch mehrere Messvorrichtungen 9 mit einem oder mehreren Sensoren angeordnet sein. Es ist auch denkbar, dass mehrere Messvorrichtungen 9 vorhanden sind, die jeweils genau einen Sensor aufweisen, der jeweils identisch ausgeführt ist und zur Messung derselben physikalischen Stoffeigenschaft bei verschiedenen Temperaturen dient.

Mit der Messvorrichtung 9 steht eine Auswertevorrichtung 12 in Signalverbindung. Die Auswertevorrichtung 12 ist dazu ausgebildet, die gemessenen physikalischen Stoffeigenschaften mit berechneten Kennwerten zu vergleichen. Die Auswertevorrichtung 12 weist insbesondere einen, insbesondere integrierten, Datenspeicher 13 auf, in dem die Kennwerte, insbesondere in Form von Kennlinienfeldern, hinterlegt sind.

Mit der Auswertevorrichtung 12 steht eine Regelungseinheit 14 in Signalverbindung.

Das LOHC-Anwendungssystem 1, die Messvorrichtung 9 und die mindestens eine Auswertevorrichtung 12 bilden ein System 15 zur Qualitätsbestimmung des LOHC. Die Regelungseinheit 14 ist dazu ausgebildet, auf Basis der bestimmten Qualitätskenngrößen als Eingangsgrößen den Betrieb des Systems 15 zu regeln.

Nachfolgend wird ein Verfahren zur Qualitätsbestimmung des LOHC näher erläutert.

In dem gezeigten Ausführungsbeispiel dient Benzyltoluol als LOHC. Mit dem System 15 wird die LOHC-Qualität anhand des Hydriergrades h und einer ersten Verunreinigungsproduktkonzentration c bestimmt, wobei als Verunreinigungsprodukt Methylfluoren angenommen wird. Insbesondere wird angenommen, dass keine weiteren Verunreinigungen vorliegen.

Die Sensoren 10, 11 dienen zur Messung von Dichte und Brechungsindex. Für die Ermittlung des Kennlinienfeldes werden fiktive Mischungen mit unterschiedlichen Hydriergraden und unterschiedlichen Verunreinigungsproduktkonzentrationen berechnet. Dabei wird insbesondere die Annahme zugrunde gelegt, dass LOHC eines beliebigen Hydriergrades durch entsprechende Mischung von vollständig beladenem LOHC, also mit einem Hydriergrad von 100 %, und mit vollständig entladenem LOHC, also mit einem Hydriergrad von 0 %, erzeugt werden kann. Entsprechend können die Mischungen für das Kennlinienfeld aus den Komponenten LOHC-H, LOHC-D und Methylfluoren gebildet werden. Für diese Komponenten sind die physikalischen Stoffeigenschaften in Tabelle 1 zusammengefasst.

| | LOHC-H | LOHC-D | Methylfluoren |
|---|---|---|---|
| Dichte @20°C [kg/m3] | 873,6 | 1002,6 | 1041,4 |
| Brechungsindex @20°C [-] | 1,4719 | 1,5608 | 1,647 |

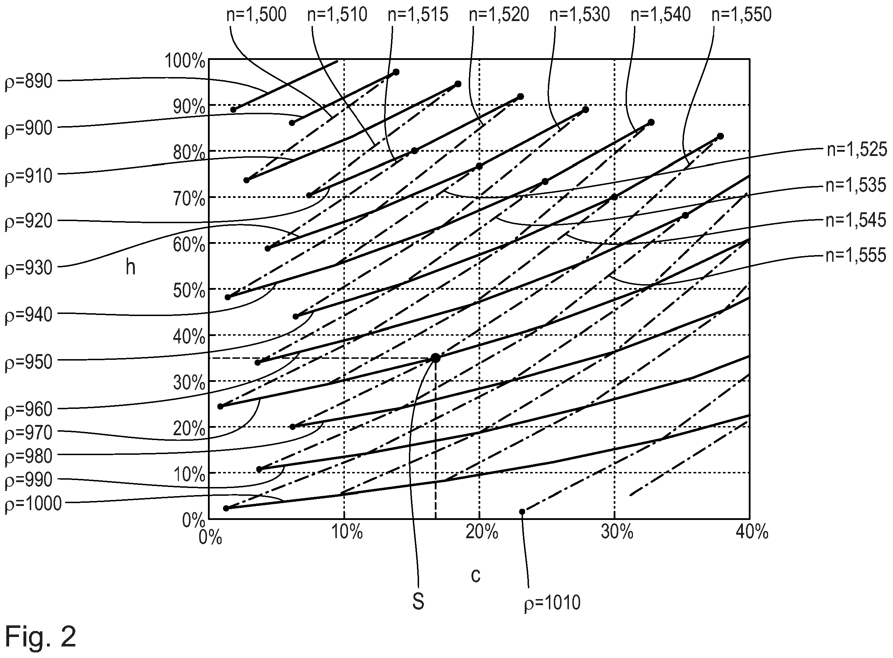

Besonders vorteilhaft ist es, wenn in einem Kennlinienfeld Mischzustände dargestellt werden, bei welchen jeweils die Dichte und der Brechungsindex konstant bleiben. Das zugehörige Kennlinienfeld ist in Fig. 2 dargestellt. In dem zugehörigen Diagramm ist entlang der Abszisse die erste Verunreinigungsproduktkonzentration c, also die Konzentration von Methylfluoren im LOHC, dargestellt. Entlang der Ordinate ist der Hydriergrad h aufgetragen. In dem Diagramm sind verschiedene Kennlinien eingezeichnet, wobei die Kennlinien in durchgezogenen Linien Mischungszustände repräsentieren, bei welchen die Dichte ρ konstant bleibt. Der jeweils zugehörige Dichtewert ist an der Kennlinie mit der Einheit kg/m³ angegeben. Entsprechend sind die mit Strichpunkt-Linien angegebenen Kennlinien repräsentativ für einen Mischungszustand, bei dem jeweils der Brechungsindex konstant bleibt. An den entsprechenden Kennlinien ist der jeweils zugehörige Wert für den Brechungsindex n angegeben.

Anhand des Kennlinienfeldes lässt sich die Anwendung des erfindungsgemäßen Verfahrens nachvollziehen. Wenn für das LOHC beispielsweise die Dichte mit 970 kg/m³ und der Brechungsindex mit 1,550 gemessen worden sind, ergibt sich der zugehörige Zustand der Mischung aus dem Schnittpunkt S der beiden Kennlinien in Fig. 2. Der Schnittpunkt S ist in Fig. 2 gekennzeichnet. Ausgehend von dem Schnittpunkt S lassen sich nun sowohl die erste Verunreinigungsproduktkonzentration für Methylfluoren zu etwa 17 % und ein Hydriergrad von etwa 35 % ablesen. Im vorliegenden Fall lassen sich die physikalischen Stoffeigenschaften, die als Qualitätskenngrößen für das LOHC dienen, unmittelbar aus dem Kennlinienfeld ablesen.

In Abhängigkeit der zur Verfügung stehenden Daten kann es erforderlich sein, dass die Qualitätskenngrößen berechnet, insbesondere interpoliert werden. Zur Verbesserung der Genauigkeit kann es vorteilhaft sein, das Kennlinienfeld in besonders relevanten Bereichen, also insbesondere bei Hydriergraden von mindestens 90 % und einer Verunreinigungsproduktkonzentration von höchstens 5 % bzw. bei einem Hydriergrad von höchstens 20 % und einer Verunreinigungsproduktkonzentration von höchstens 5 % zu erfassen und in diesem Bereich mit einer höheren Auflösung, also mit mehr Kennlinien als in Fig. 2 dargestellt, zu erfassen. Dadurch wird die Genauigkeit bei der Bestimmung der Qualitätskenngrößen verbessert.

Es kann zudem vorteilhaft sein, für die Bestimmung des Kennlinienfeldes, insbesondere gängige, teilhdydrierte Spezies des Wasserstoffträgermaterials, insbesondere bei Benzyltoluol die Spezies H0-BT, H6-BT und H12-BT, sowie gängige Verunreinigungsprodukte zu berücksichtigen. Dabei steht H0-BT für die entladene Form des Benzyltoluols und H12-BT für die beladene Form des Benzyltoluols, also Perhydro-Benzyltoluol. H6-BT steht für die teilweise beladene Form des Benzyltoluols, bei dem sechs Wasserstoffatome an dem Benzyltoluol-Monekül chemisch gebunden sind.

## Patentansprüche

1. Verfahren zur Qualitätsbestimmung eines flüssigen organischen Wasserstoffträgermaterials umfassend die Verfahrensschritte
- Nutzen des flüssigen organischen Wasserstoffträgermaterials in einem LOHC-Anwendungssystem (1),
- Messen mindestens zwei physikalischer Stoffeigenschaften des flüssigen organischen Wasserstoffträgermaterials in dem LOHC-Anwendungssystem (1),
- Vergleichen der gemessenen physikalischen Stoffeigenschaften mit Kennwerten für das flüssige organische Wasserstoffträgermaterial bei unterschiedlich großen Hydriergraden und bei unterschiedlich großen Verunreinigungsproduktkonzentrationen,
- Bestimmen des Hydriergrades (h) und einer ersten Verunreinigungsproduktkonzentration (c) als Qualitätskenngrößen des flüssigen organischen Wasserstoffträgermaterials.

2. Verfahren gemäß Anspruch 1, **gekennzeichnet durch** Messen mindestens einer dritten physikalischen Stoffeigenschaft und Bestimmen mindestens einer dritten Qualitätskenngröße, insbesondere einer zweiten Verunreinigungsproduktkonzentration.

3. Verfahren gemäß einem der vorstehenden Ansprüche, **dadurch gekennzeichnet, dass** die physikalischen Stoffeigenschaften an unterschiedlichen Stellen im LOHC-Anwendungssystem (1) gemessen werden, insbesondere an unterschiedlichen Stellen entlang einer Strömungsrichtung des flüssigen organischen Wasserstoffträgermaterials im LOHC-Anwendungssystem (1).

4. Verfahren gemäß einem der vorstehenden Ansprüche, **dadurch gekennzeichnet, dass** die physikalischen Stoffeigenschaften, insbesondere zumindest teilweise, in mindestens einer Bypass-Leitung (8) des LOHC-Anwendungssystems (1) gemessen werden.

5. Verfahren gemäß einem der vorstehenden Ansprüche, **dadurch gekennzeichnet, dass** unterschiedliche physikalische Stoffeigenschaften in gemessen werden, insbesondere Dichte und Brechungsindex.

6. Verfahren gemäß einem der vorstehenden Ansprüche, **dadurch gekennzeichnet, dass** zumindest eine physikalische Stoffeigenschaft bei unterschiedlichen Temperaturen gemessen wird.

7. Verfahren gemäß einem der vorstehenden Ansprüche, **dadurch gekennzeichnet, dass** das flüssige organische Wasserstoffträgermaterial Benzyltoluol ist.

8. Verfahren gemäß einem der vorstehenden Ansprüche, **dadurch gekennzeichnet, dass** das mindestens eine Verunreinigungsprodukt Methylfluoren ist.

9. Verfahren gemäß einem der vorstehenden Ansprüche, **dadurch gekennzeichnet, dass** die Menge an Verunreinigungsprodukten bestimmt wird.

10. Verfahren gemäß einem der vorstehenden Ansprüche, **gekennzeichnet durch** Nutzen der Qualitätskenngrößen für den Betrieb des LOHC-Anwendungssystems (1), insbesondere
- als Prozessregelgröße,
- zur Überwachung der Katalysatorstabilität bei der Hydrierung und/oder Dehydrierung des flüssigen organischen Wasserstoffträgermaterials,
- zur Qualitätskontrolle in der LOHC-Logistik und/oder
- zur Bewertung des Prozesszustands.

11. System zur Qualitätsbestimmung eines flüssigen organischen Wasserstoffträgermaterials umfassend
a. ein LOHC-Anwendungssystem (1), in dem das flüssige organische Wasserstoffträgermaterial nutzbar ist,
b. eine einen Sensor (10, 11) aufweisende Messvorrichtung (9) zum Messen mindestens zwei physikalischer Stoffeigenschaften des flüssigen organischen Wasserstoffträgermaterials in dem LOHC-Anwendungssystem (1),
c. mindestens eine Auswertevorrichtung (12), die dazu ausgebildet ist, die gemessenen physikalischen Stoffeigenschaften mit Kennwerten für das flüssige organische Wasserstoffträgermaterial bei unterschiedlich großen Hydriergraden und bei unterschiedlich großen Verunreinigungsproduktkonzentrationen zu vergleichen sowie den Hydriergrad (h) und eine erste Verunreinigungsproduktkonzentration (c) als Qualitätskenngrößen des flüssigen organischen Wasserstoffträgermaterials zu bestimmen.

12. System gemäß Anspruch 11, **gekennzeichnet durch** eine mit der Auswerteeinheit (12) in Signalverbindung stehende Regelungseinheit (14), die dazu ausgebildet ist, auf Basis der Qualitätskenngrößen als Eingangsgrößen für die Regelung den Betrieb des Systems (15) zu regeln.
